(19) Europäisches Patentamt
European Patent Office
Office européen des brevets

(11) Veröffentlichungsnummer: **0 000 567**
**B1**

(12) **EUROPÄISCHE PATENTSCHRIFT**

(21) Anmeldenummer: **78100475.9**

(22) Anmeldetag: **21.07.78**

(51) Int. Cl.³: **C 07 C 49/603,**
C 07 C 49/613,
C 07 C 45/27, //
C 08 L 23/06

(54) **Verfahren zur Herstellung von 2,6-Di-tert.-alkyl-4-alkyliden-2,5-cyclohexadien-onen**

(30) Priorität: **29.07.77 DE 2734239**

(43) Veröffentlichungstag der Anmeldung:
**07.02.79 Patentblatt 79/03**

(45) Bekanntmachung des Hinweises auf die Patenterteilung:
**03.09.80 Patentblatt 80/18**

(84) Benannte Vertragsstaaten:
**BE CH DE FR GB LU NL**

(56) Entgegenhaltungen:
**DE - A - 2 363 464**
**US - A - 3 923 767**
**Chemical Abstracts, vol. 86, 1977**
**Columbus Ohio "Synth. Commun" 1976.6 (4), Seiten 305—8**

(73) Patentinhaber: **Bayer AG**
**Zentralbereich Patente, Marken und Lizenzen**
**D - 5090 Leverkusen 1, Bayerwerk (DE)**

(72) Erfinder: **Roos, Ernst, Dr.**
**Am Geus Garten 6**
**D - 5068 Odenthal (DE)**
**Hugl, Erika, Dr.**
**Elisabeth-Langgaesser-Strasse 19**
**D - 5090 Leverkusen 1 (DE)**

**EP 0 000 567 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Herstellung von 2,6-Di-tert.-alkyl-4-alkyliden-2,5-cyclohexadien-onen

Die Erfindung betrifft ein Verfahren zur Herstellung von 2,6-Di-tert.-alkyl-4-alkyliden-2,5-cyclohexadien-onen (Chinon-methide).

2,6-Di-tert.-alkyl-4-alkyliden-2,5-cyclohexadien-one sind bekannt und können in einem mehrstufigen Verfahren durch Oxidation aus 2,4,6-Trialkylphenolen hergestellt worden (US-PS 3 923 767).

Es wurde ein Verfahren zur Herstellung von 2,6-Di-tert.-alkyl-4-alkyliden-2,5-cyclohexadien-onen der Formel

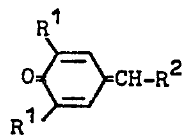

(I)

gefunden, bei dem man Bis-(3,5-di-tert.-alkyl-4-hydroxy-benzyl)-sulfide der Formel

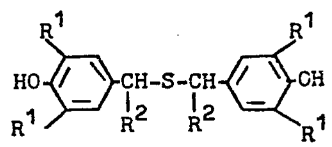

(II)

worin

$R^1$ tertiäres Alkyl und

$R^2$ Wasserstoff, geradkettiges oder verzweigtes Alkyl, Cyloalkyl, Cycloalkenyl, Aralkyl oder Aryl, bedeutet,

mit einem Überschuss von Oxiden, Hydroxiden, Carbonaten oder basischen Salzen von Schwermetallen in der zweiwertigen Oxidationsstufe bei 50 bis 200°C in einem inerten Lösungsmittel aus der Gruppe der niederen Alkohole, der Ether, der Ketone, der Säureamide oder der Kohlenwasserstoffe umsetzt.

Das erfindungsgemäße Verfahren kann durch die folgende Reaktionsgleichung erläutert werden:

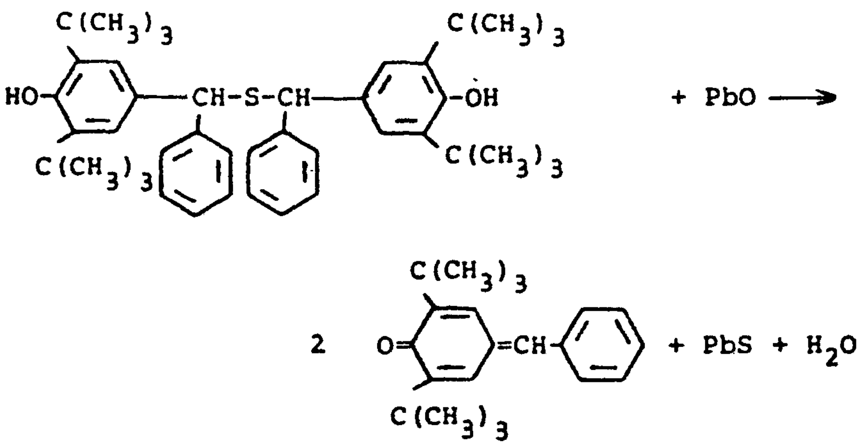


Als tertiäre Alkylreste ($R^1$) für das erfindungsgemäße Verfahren seien Kohlenwasserstoffreste genannt, die die benachbarte Hydroxylgruppe behindern. Vorzugsweise seien Kohlenwasserstoffreste, wie tert.-Butyl, tert.-Pentyl, tert.-Hexyl, tert.-Heptyl, tert.-Octyl, insbesondere tert.-Butyl, genannt.

Als Alkyl ($R^2$) seien geradkettige oder verzweigte Kohlenwasserstoffreste mit 1 bis 12 Kohlenstoffatomen, bevorzugt mit 1 bis 6 Kohlenstoffatomen genannt. Beispielhaft seien die folgenden Reste genannt: Methyl, Äthyl, Propyl, Isopropyl, Butyl, Isobutyl, tert.-Butyl, Pentyl, Isopentyl, Hexyl, Isohexyl, Heptyl, Isoheptyl, Octyl, Isooctyl, Nonyl, Isononyl, Decyl, Isodecyl, Dodecyl, Isododecyl.

Als Cycloalkyl ($R^2$) kommen besonders gegebenenfalls durch nieder-Alkyl substituierte fünfgliedrige und sechsgliedrige Kohlenwasserstoffringe in Frage. Beispielsweise seien genannt: Cyclopentyl, Cyclohexyl, Methylcyclohexyl.

Als Cycloalkenyl ($R^2$) kommen besonders gegebenenfalls durch nieder-Alkyl substituierte fünfgliedrige und sechsgliedrige ungesättigte Kohlenwasserstoffringe in Frage. Beispielsweise seien genannt: Cyclopentenyl, Cyclohexenyl, Methylcyclohexenyl.

Die Aralkylreste ($R^2$) können in ihrem aliphatischen Teil 1 bis 4 Kohlenstoffatome haben und in ihrem aromatischen Teil einen gegebenenfalls durch Chloratome oder Methylgruppen substituierten Rest aus der Benzolreihe, wie Phenyl, darstellen. Beispielsweise seien der Benzylrest und der gegenbenenfalls durch Chloratome oder Methylgruppen substituierte Benzylrest genannt.

Arylreste ($R^2$) können carbocyclische Reste aus der Benzolreihe sein, die gegebenenfalls durch Halogen nieder-Alkyl oder Phenyl substituiert sein können. Als Halogensubstituenten seien beispielsweise Fluor, Chlor, Brom oder Jod genannt. Als Alkylsubstituenten der Arylreste seien beispielsweise Methyl, Äthyl, Isopropyl oder tert.-Butyl genannt. Beispielsweise seien als Arylreste gegebenenfalls substituierte Phenylreste oder Diphenylreste genannt.

Die Bis-(3,5-di-tert.-alkyl-4-hydroxy-benzyl)-sulfide (II) sind bekannt und können durch Umsetzung von Phenolen und Aldehyden und Alkalisulfiden hergestellt werden (DT—OS 2 363 464).

Beispielhaft seien die folgenden Ausgangsverbindungen (II) genannt:

Bis - [3,5 - di - tert. - butyl - 4 - hydroxy - ($\alpha$ - isopropyl) - benzyl] - sulfid
Bis - [3,5 - di - tert. - butyl - 4 - hydroxy - ($\alpha$ - tert. - butyl) - benzyl] - sulfid
Bis - [3,5 - di - tert. - butyl - 4 - hydroxy - ($\alpha$ - cyclohexen - $\Delta^3$ - yl) - benzyl] - sulfid
Bis - [3,5 - di - tert. - butyl - 4 - hydroxy - ($\alpha$ - cyclohexyl) - benzyl] - sulfid
Bis - [3,5 - di - tert. - butyl - 4 - hydroxy - ($\alpha$ - phenyl) - benzyl] - sulfid
Bis - [3,5 - di - tert. - butyl - 4 - hydroxy - ($\alpha$ - p - diphenyl) - benzyl] - sulfid
Bis - [3,5 - di - tert. - butyl - 4 - hydroxy - ($\alpha$ - o - chlor - phenyl) - benzyl] - sulfid
Bis - [3,5 - di - tert. - butyl - 4 - hydroxy - ($\alpha$ - m - chlor - phenyl) - benzyl] - sulfid
Bis - [3,5 - di - tert. - butyl - 4 - hydroxy - ($\alpha$ - p - chlor - phenyl) - benzyl] - sulfid
Bis - [3,5 - di - tert. - butyl - 4 - hydroxy - ($\alpha$ - o,p - dichlor - phenyl) - benzyl] - sulfid
Bis - [3,5 - di - tert. - butyl - 4 - hydroxy - ($\alpha$ - o,o - dichlor - phenyl) - benzyl] - sulfid
Bis - [3,5 - di - tert. - butyl - 4 - hydroxy - ($\alpha$ - o - tolyl) - benzyl] - sulfid
Bis - [3,5 - di - tert. - butyl - 4 - hydroxy - ($\alpha$ - m - tolyl) - benzyl] - sulfid
Bis - [3,5 - di - tert. - butyl - 4 - hydroxy - ($\alpha$ - p - tolyl) - benzyl] - sulfid

Basische Verbindungen von Schwermetallen in der zweiwertigen Oxidationsstufe für das erfindungsgemäße Verfahren können im allgemeinen solche des Bleies und des Quecksilbers, bevorzugt solche des Bleies, sein.

Basische Verbindungen sind beispielsweise die Oxide, die Hydroxide oder die Carbonate.

Beispielhaft seien die folgenden Verbindungen genannt: Blei-(II)-oxid, Blei-(II,IV)-oxid (Mennige), Blei-(II)-carbonat, basisches Bleicarbonat ($2\ PbCO_3 \cdot Pb(OH)_2$ = Bleiweiß) Bleioxid-carbonate vom Typ $3PbO \cdot 5PbCO_3$, $PbO \cdot PbCO_3$ und $2PbO \cdot PbCO_3$, basisches Bleiacetat und Quecksilber-(II)-oxid.

Besonders bevorzugt ist die Verwendung von basischem Bleicarbonat.

Die erfindungsgemäß eingesetzten basischen Verbindungen von Schwermetallen können in stöchiometrischen Mengen, bezogen auf das Bis - [3,5 - di - tert. - alkyl - 4 - hydroxy - benzyl) - sulfid eingesetzt werden. Selbstverständlich können die Schwermetall-Verbindungen auch in geringerer als der stöchiometrischen Menge eingesetzt werden. Bevorzugt wird ein Überschuß der Schwermetall-Verbindungen von etwa 100 bis 110 Mol-%, bezogen auf das Bis-(3,5-di-tert.-alkyl-4-hydroxybenzyl)-sulfid, eingesetzt.

Das erfindungsgemäße Verfahren kann im Temperaturbereich von etwa 50 bis etwa 200°C, vorzugsweise im Temperaturbereich von etwa 100 bis etwa 150°C, durchgeführt werden.

Das erfindungsgemäße Verfahren kann sowohl bei Normaldruck als auch bei Unterdruck oder Überdruck durchgeführt werden. Die Einstellung eines bestimmten Druckes ist nicht wesentlich für das erfindungsgemäße Verfahren. Zur Einhaltung einer bestimmten Reaktionstemperatur kann gegebenenfalls bei Verwendung von hochsiedenden Lösungsmitteln bei Unterdruck oder bei Verwendung von niedrig siedenden Lösungsmitteln bei Überdruck gearbeitet werden. Bevorzugt wird das erfindungsgemäße Verfahren bei Normaldruck durchgeführt.

Das erfindungsgemäße Verfahren wird in einem Lösungsmittel durchgeführt, das unter den erfindungsgemäßen Bedingungen nicht verändert wird.

Das Lösungsmittel gehört zur Gruppe der niederen Alkohole, der Äther, der Ketone, der Säureamide oder der Kohlenwasserstoffe angehören.

Niedere Alkohole für das erfindungsgemäße Verfahren können einen geradkettigen oder verzweigten Kohlenwasserstoffrest ($C_1$—$C_4$) haben. Beispielsweise seien Methanol, Äthanol, Isopropanol oder die verschiedenen isomeren Butanole genannt.

Äther für das erfindungsgemäße Verfahren können offenkettige oder cyclische Äther sein. Beispielsweise seien Dioxan, Tetrahydrofuran, Äthylenglykolmono- oder -dialkyläther genannt.

Die Ketone für das erfindungsgemäße Verfahren können aliphatische ($C_1$—$C_4$) oder aromatische Reste (Phenyl) tragen. Beispielsweise seien Aceton, Methyläthylketon, Diäthylketon oder Acetophenon genannt.

Säureamide für das erfindungsgemäße Verfahren können am Stickstoffatom unsubstituiert oder durch niedere Alkylreste, beispielsweise Methyl, substituiert sein. Beispielsweise seien Formamid, Methylformamid, Dimethylformamid oder Dimethylacetamid genannt.

Die Kohlenwasserstoffe für das erfindungsgemäße Verfahren können der aliphatischen oder der aromatischen Reihe angehören und gegebenenfalls durch Halogen, beispielsweise Chlor oder niedere Alkylgruppen, beispielsweise Methyl, substituiert sein. Beispielsweise seien Benzinfraktionen mit 6 bis 10 Kohlenstoffatomen, Cylohexan, Benzol, Chlorbenzol, Toluol oder Xylol genannt.

In einer bevorzugten Reaktionsweise können Lösungsmittel, die nicht mit Wasser mischbar sind und mit denen eine azeotrope Destillation des bei dem Verfahren entstehenden Reaktionswassers möglich ist, eingesetzt werden. Diese Art der Durchführung des erfindungsgemäßen Verfahren läßt den Fortgang und den endpunkt der Umsetzung anhand der abgegebenen Wassermenge erkennen. Geeignete Lösungsmittel hierfür sind beispielsweise Benzol, Chlorbenzol, Toluol oder Xylol.

Vorzugsweise wird das erfindungsgemäße Verfahren in Xylol beim Siedepunkt des Lösungsmittels durchgeführt.

Beispielsweise kann das erfindungsgemäße Verfahren wie folgt durchgeführt werden.

Das als Ausgangsverbindung eingesetzte Bis-(3,5-di-tert.-alkyl-4-hydroxy-benzyl)-sulfid, das Lösungsmittel und die erforderliche Menge der basischen Schwermetall-Verbindung in der zweiwertigen Oxidationsstufe werden in einem Reaktionsgefäß vorgelegt, das mit einem Rührer, einem Thermometer, einem Rückflußkühler und gegebenenfalls einem Wasserabscheider ausgestattet ist.

Dann wird das Reaktionsgemisch unter Rühren etwa 5 bis 12 Stunden bei der Reaktionstemperatur gehalten. In einer bevorzugten Verfahrensweise wird die Reaktionsmischung bei Verwendung von Xylol als Lösungsmittel solange auf die Siedetemperatur des Xylols erhitzt, bis die Wasserabscheidung beendet ist. Hierfür wird ein Reaktionsgefäß mit Wasserabscheider benötigt. Die abgekühlte Reaktionsmischung wird sodann von den anorganischen Bestandteilen abfiltriert und das erfindungsgemäß dargestellte Chinonmethid nach üblichen Methoden gewonnen. Solche üblichen Methoden sind z.B.: das Abdampfen des Lösungsmittels im Vakuum und die Gewinnung des Endproduktes als Kristallmasse oder als destillierbares Öl oder die Einengung der reaktionslösung und die Kristallisation des Endproduktes aus der eingeengten Lösung oder die direkte Weiterverwendung der Reaktionslösung des Endproduktes. Die Umsetzung und die Aufarbeitung nach dem erfindungsgemäßen Verfahren können gegebenenfalls auch unter Inertgas, z.B. unter Stickstoff, vorgenommen werden. Diese Maßnahme ist abhängig von der Stabilität der eingesetzten Ausgangsverbindung.

Es ist bekannt, daß die als Ausgangsverbindungen eingesetzten Bis-(3,5-di-tert.-alkyl-4-hydroxybenzyl)sulfide mit Alkalihydroxyden 2,6-Di-tert.-alkyl-4-alkenyl-phenole ergeben (DT—OS 2 363 464). Es ist daher überraschend, daß unter den erfindungsgemäß besischen Bedingungen Chinon-methide entstehen.

Vorteilhafterweise können die Chinonmethide nach dem erfindungsgemäßen Verfahren in einfacher Weise mit großen Ausbeuten in einem Reaktionsschritt hergestellt werden.

Beispielsweise lassen sich nach dem erfindungsgemäßen Verfahren Chinonmethide der Formel

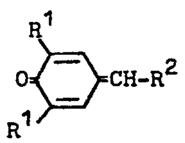

(I)

worin

$R^1$ tertiäres Alkyl und

$R^2$ Wasserstoff, geradkettiges oder verzweigtes Alkyl, Cycloalkyl, Cycloalkenyl, Aralkyl oder Aryl bedeutet,

herstellen.

Die Chinon-methide können als Additive bei der Äthylen-Polymerisation eingesetzt werden und bewirken eine verbesserte Transparenz des hergestellten Polyäthylens (US—PS 3 923 767).

## Beispiel 1

Herstellung von 2,6-Di-tert.-butyl-4-isobutyliden-2,5-cyclohexadien-on:

277 g (0,5 Mol) Bis - [3,5 - di - tert. - butyl - 4 - hydroxy - ($\alpha$ - isopropyl) - benzyl] - sulfid, 1000 ml Xylol und 200 g basisches Bleicarbonat werden 5 Stunden am Wasserabscheider zum Sieden erhitzt, wobei sich 10 ml Wasser abscheiden. Die gelbe Xylollösung wird von den anorganischen Bestandteilen abfiltriert und im Vakuum zur Trockene eingedampft. Der Rückstand kristallisiert beim Erkalten zu einer gelben Kristallmasse.

Ausbeute: 251 g; entspr. 96,5% der theoretischen Ausbeute, gelbe Kristalle, Fp. 62 bis 64°C, Kp 0,05 mbar/112°C.

## Beispiel 2

Herstellung von 2,6 - Di - tert. - butyl - 4 - cyclohexen - $\Delta^3$ - yl - methyliden - 2,5 - cyclohexadien - on:

252 g (0,4 Mol) Bis - [3,5 - di - tert. - butyl - 4 - hydroxy - ($\alpha$ - cyclohexen - $\Delta^3$ - yl) - benzyl] - sulfid, 1000 ml Xylol und 300 g basisches Bleicarbonat werden 12 Stunden am Wasserabscheider zum Sieden erhitzt, wobei sich 8 ml Wasser abscheiden. Nach Abfiltrieren der anorganischen bestandteile wird die gelbe Xylollösung im Vakuum zur Trockene eingedampft. Als Rückstand bleibt ein viskoses, rotbraunes Öl zurück.

Ausbeute: 207 g; entspr. 86,7% der Theorie,
Kp 0,05 mbar/145°C.

Beispiel 3

Herstellung von 2,6 - Di - tert. - butyl - 4 - benzyliden - 2,5 - cyclohexadien - on:

311 g (0,5 Mol) Bis - [3,5 - di - tert. - butyl - 4 - hydroxy - ($\alpha$ - phenyl) - benzyl] - sulfid, 1000 ml Xylol und 190 g basisches Bleicarbonat werden 10 Stunden am Wasserabscheider zum Sieden erhitzt, wobei sich 10 ml Wasser abscheiden. Nach Abfiltrieren der anorganischen Bestandteile wird das Filtrat im Vakuum zur Trockene eingedampft. Der Rückstand kristallisiert beim Erkalten zu einer gelben Kristallmasse.

Ausbeute: 272 g = 92,5% der Theorie

gelbe Kristalle Fp. 71 bis 76°C

Kp 0,1 mbar/147°C.

Beispiel 4

Herstellung einer Lösung von 2,6 - Di - tert. - butyl - 4 - methyliden - 2,5 - cyclohexadien - on:

47 g (0,1 Mol) Bis - (3,5 - di - tert. - butyl - 4 - hydroxy - benzyl) - sulfid, 300 ml Xylol und 40 g basisches Bleicarbonat werden unter Stickstoff 6 Stunden am Wasserabscheider gekocht, wobei 2 ml Wasser übergehen. Durch Abfiltrieren von den anorganischen Bestandteilen erhält man eine gelbe Xylollösung des 3,5 - Di - tert. - butyl - p - chinon - methids.

Die dargestellte 4-Methyliden-Verbindung ist nur in verdünnter Lösung stabil und wird durch Aufnahme des NMR-Spektrums (Banden bei 3,18, 4,38 und 8,73 $\tau$) charakterisiert.

Beispiele 5—15

Die Beispiele 5 bis 15 werden nach der Arbeitsvorschrift des Beispiels 1 in Gegenwart von basischem Bleicarbonat durchgeführt. Die Reaktionsparameter sind in Tabelle 1 angeführt.

TABELLE 1

| Beispiel Nr. | Ausgangsprodukt | Endprodukt | Ausbeute [%] | Eigenschaften des Endprodukts | |
|---|---|---|---|---|---|
| 5 | Bis-[3,5-di-tert.-butyl-4-hydroxy-(α-tert.-butyl)-benzyl]-sulfid | 2,6-Di-tert.-butyl-4-(β,β-dimethyl)-propyliden-2,5-cyclohexadien-on | 81 | gelbe Kristalle | Fp. 40–42 °C |
| 6 | Bis-[3,5-di-tert.-butyl-4-hydroxy-(α-cyclohexyl)-benzyl]-sulfid | 2,6-Di-tert.-butyl-4-cyclohexyl-methyliden-2,5-cyclohexadien-on | 83 | gelbe Kristalle | Fp. 42–44 °C |
| 7 | Bis[3,5-di-tert.-butyl-4-hydroxy-(α-p-diphenyl)-benzyl]-sulfid | 2,6-Di-tert.-butyl-4-p-diphenyl-methyliden-2,5-cyclohexadien-on | 89 | gelbe Kristalle | Fp. 135–137 °C |
| 8 | Bis-[3,5-di-tert.-butyl-4-hydroxy-(α-o-chlor-phenyl)-benzyl]-sulfid | 2,6-Di-tert.-butyl-4-o-chlor-phenyl-methyliden-2,5-cyclohexadien-on | 91 | gelbe Kristalle | Fp. 109–111 °C |
| 9 | Bis-[3,5-di-tert.-butyl-4-hydroxy-(α-m-chlor-phenyl)-benzyl]-sulfid | 2,6-Di-tert.-butyl-4-m-chlor-phenyl-methyliden-2,5-cyclohexadien-on | 97 | zähes, rotes Öl | Kp. 150 °C/0,1 mbar |
| 10 | Bis-[3,5-di-tert.-butyl-4-hydroxy-(α-p-chlor-phenyl)-benzyl]-sulfid | 2,6-Di-tert.-butyl-4-p-chlor-phenyl-methyliden-2,5-cyclohexadien-on | 96,5 | gelbe Kristalle | Fp. 132–134 °C |
| 11 | Bis-[3,5-di-tert.-butyl-4-hydroxy-(α-o,p-dichlor-phenyl)-benzyl]-sulfid | 2,6-Di-tert.-butyl-4-o,p-dichlor-phenyl-methyliden-2,5-cyclohexadien-on | 83 | gelbe Kristalle | Fp. 163–165 °C |
| 12 | Bis-[3,5-di-tert.-butyl-4-hydroxy-(α-o,o-dichlor-phenyl)-benzyl]-sulfid | 2,6-Di-tert.-butyl-4-o,o-dichlor-phenyl-methyliden-2,5-cyclohexadien-on | 92 | gelbe Kristalle | Fp. 112–114 °C |
| 13 | Bis-[3,5-di-tert,-butyl-4-hydroxy-(α-o-tolyl)-benzyl]-sulfid | 2,6-Di-tert.-butyl-4-o-tolyl-methyliden-2,5-cyclohexadien-on | 90 | gelbe Kristalle | Fp. 99–102 °C |
| 14 | Bis-[3,5-di-tert.-butyl-4-hydroxy-(α-m-tolyl)-benzyl]-sulfid | 2,6-Di-tert.-butyl-4-m-tolyl-methyliden-2,5-cyclohexadien-on | 98 | gelbe Kristalle | Fp. 55–57 °C |
| 15 | Bis-[3,5-di-tert.-butyl-4-hydroxy-(α-p-tolyl)-benzyl]-sulfid | 2,6-Di-tert,-butyl-4-p-tolyl-methyliden-2,5-cyclohexadien-on | 91 | gelbe Kristalle | Fp. 143 °C |

Beispiel 16

55,4 g (0,1 Mol) Bis - [3,5 - di - tert. - butyl - 4 - hydroxy - ($\alpha$ - isopropyl) - benzyl] - sulfid, 300 ml Xylol und 40 g Blei-II-oxid werden 10 Stunden am Wasserabscheider zum Sieden erhitzt, wobei etwa 2 ml Wasser abgeschieden werden. Die Xylollösung wird von den organischen Bestandteilen abfiltriert und im Vakuum zur Trockene eingedampft. Der Rückstand kristallisiert beim Erkalten zu einer gelben Kristallmasse.

Ausbeute: 22,8 g = 44% der Theorie,
gelbe Kristalle, Fp. 62°C.

Beispiel 17

63 g (0,1 Mol) Bis - [3,5 - di - tert. - butyl - 4 - hydroxy - ($\alpha$ - cyclohexen - $\Delta^3$ - yl - benzyl] - sulfid, 300 ml Xylol und 67 g (0,3 Mol) Blei-II-oxid wurden 25 Stunden am Wasserabscheider zum Sieden erhitzt, wobei sich etwa 2 ml Wasser abscheiden. Nach Filtrieren und Eindampfen bleibt ein Öl zurück.

Ausbeute: 44 g = 74% der Theorie,
viskoses braunes Öl, Kp. 0,05 mbar/140—150°C.

Beispiel 18

63 g (0,1 Mol) Bis - [3,5 - di - tert. - butyl - 4 - hydroxy - ($\alpha$ - cyclohexen - $\Delta^3$ - yl - benzyl] - sulfid, 300 Dioxan und 67 g (0,3 Mol) Blei-II-oxid werden 25 Stunden unter Rückfluß erhitzt. Nach Filtrieren und Eindampfen bleibt ein Öl zurück.

Ausbeute: 21 g = 36,5% der Theorie,
viskoses braunes Öl, Kp. 0,06 mbar/138—148°C.

Beispiel 19

62,3 g (0,1 Mol) Bis - [3,5 - di - tert. - butyl - 4 - hydroxy - ($\alpha$ - phenyl) - benzyl] - sulfid, 300 ml Xylol und 67 g (0,3 Mol) Blei-II-oxid werden 10 Stunden am Wasserabscheider zum Sieden erhitzt, wobei etwa 2 ml Wasser abgeschieden werden. Nach Filtrieren und Eindampfen kristallisiert der Rückstand durch.

Ausbeute: 41 g = 70% der Theorie,
gelbe Kristalle, Fp. 73—75°C.

Beispiel 20

Man arbeitet wie in Beispiel 19, verwendet jedoch 65 g Quecksilber-II-oxid anstatt Blei-II-oxid.

Ausbeute: 41 g = 70% der Theorie,
gelbe Kristalle, Fp. 73—75°C.

Beispiel 21

Wenn man in Beispiel 3 anstelle eines Wasserabscheiders einen normalen Rückflußkühler verwendet, erhält man unter sonst gleichen Bedingungen ein rotes Öl, welches erst nach längerem Stehen erstarrt. Nach Destillation bei Kp. 0,09/140—150°C erhält man daraus das gleiche Produkt in einer Ausbeute von 72% der Theorie.

Beispiel 22

Es wird wie im Beispiel 3 verfahren, jedoch wird anstelle von Xylol Toluol als Lösungsmittel eingesetzt. Es wird solange zum Sieden erhitzt, bis sich 10 ml Wasser abgeschieden haben. Gleiche Ausbeute wie im Beispiel 3.

Beispiel 23

Es wird wie im Beispiel 3 verfahren, jedoch wird anstelle von Xylol Benzol als Lösungsmittel eingesetzt. Es wird solange zum Sieden erhitzt, bis sich 10 ml Wasser abgeschieden haben. Gleiche Ausbeute wie im Beispiel 3.

**Patentansprüche**

1. Verfahren zur Herstellung von 2,6-Di-tert.-alkyl-4-alkyliden-2,5-cyclohexadien-onen der Formel

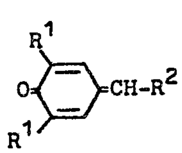

dadurch gekennzeichnet, daß man Bis-(3,5-di-tert.-alkyl-4-hydroxybenzyl)-sulfide der Formel

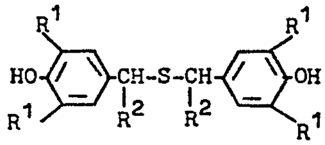

worin

$R^1$ tertiäres Alkyl und

$R^2$ Wasserstoff, ein geradkettiges oder verzweigtes Alkyl, Cycloalkyl, Cycloalkenyl, Aralkyl oder Aryl

bedeutet,

mit einem Überschuß von Oxiden, Hydroxiden, Carbonaten oder basischen Salzen von Schwermetallen in der zweiwertigen Oxidationsstufe bei 50 bis 200°C in einem inerten Lösungsmittel aus der Gruppe der niederen Alkohole, der Ether, der Ketone, der Säureamide oder der Kohlenwasserstoffe umsetzt.

2. Verfahren nach Anspruch 1, dadurch gekennzeichnet, daß man basisches Bleicarbonat verwendet.

3. Verfahren nach Anspruch 1 und 2, dadurch gekennzeichnet, daß man die Umsetzung in siedendem Xylol unter gleichzeitiger Entfernung des bei der Umsetzung entstehenden Wassers durch azeotrope Destillation durchführt.

## Claims

1. Process for the preparation of 2,6-di-tert.-alkyl-4-alkylidene-2,5-cyclohexadien-ones of the formula

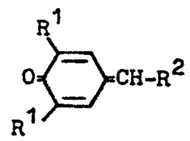

characterised in that bis-(3,5-di-tert.-alkyl-4-hydroxybenzyl) sulphides of the formula

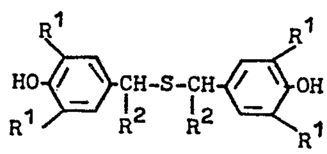

wherein

$R^1$ denotes tertiary alkyl and

$R^2$ denotes hydrogen, a straight-chain or branched alkyl, cycloalkyl, cycloalkenyl, aralkyl or aryl, are reacted with an excess of oxides, hydroxides, carbonates or basic reacting salts of heavy metals in the divalent oxidation stage at from 50 to 200°C in inert solvents from the group of the lower alcohols, the ethers, the ketones, the acid amides or the hydrocarbons.

2. Process according to Claim 1, characterised in that basic lead carbonate is used.

3. Process according to Claim 1 to 2, characterised in that the reaction is carried out in boiling xylene, the water formed during the reaction being simultaneously removed by azeotropic distillation.

## Revendications

1. Procédé de production de 2,6-di-tertio-alkyl-4-alkylidène-2,5-cyclohexadiénones de formule

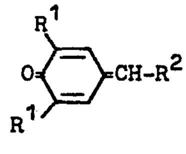

caractérié en ce qu'il consiste à faire réagir des sulfures de bis-(3,5-di-tertio-alkyl-4-hydroxybenzyle) de formule:

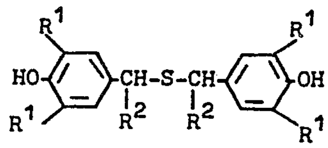

(dans laquelle $R^1$ est un groupe alkyle tertiaire et $R^2$ est un atome d'hydrogène, un groupe alkyl à chaîne droite ou ramifiée, un groupe cycloalkyle, un groupe cycloalcényle, un groupe aralkyle ou un groupe aryle), avec un excédent des oxydes, des hydroxydes, des carbonates ou des sels basiques de métaux lourds au degré d'oxydation de valence deux à 50—200°C dans des solvants inertes du groupe des alcools inférieurs, des éthers, des cétones, des amides d'acide ou des hydrocarbures.

2. Procédé suivant la revendication 1, caractérisé en ce qu'il consiste à utilisier le carbonate de plomb basique.

3. Procédé suivant l'une quelconque des revendications 1 à 2, caractérisé en ce que la réaction est conduite dans du xylène bouillant, avec élimination simultanée par distillation azéotropique de l'eau produite dans la réaction.